(19) Europäisches Patentamt
European Patent Office
Office européen des brevets

(11) EP 1 960 513 B1

(12) **EUROPEAN PATENT SPECIFICATION**

(45) Date of publication and mention of the grant of the patent:
**23.06.2010 Bulletin 2010/25**

(21) Application number: **06844973.5**

(22) Date of filing: **08.12.2006**

(51) Int Cl.:
***C12N 5/02*** *(2006.01)* ***D04H 13/00*** *(2006.01)*

(86) International application number:
**PCT/US2006/046740**

(87) International publication number:
**WO 2007/078568 (12.07.2007 Gazette 2007/28)**

(54) **NON-WOVEN FABRIC FOR BIOMEDICAL APPLICATION BASED ON POLY (ESTER-AMIDE)S**

VLIESFASER FÜR EINE BIOMEDIZINISCHE ANWENDUNG BASIEREND AUF POLY(ESTERAMID) EN

TISSU NON TISSE POUR UNE APPLICATION BIOMEDICALE A BASE DE POLYESTERAMIDES

(84) Designated Contracting States:
**AT BE BG CH CY CZ DE DK EE ES FI FR GB GR HU IE IS IT LI LT LU LV MC NL PL PT RO SE SI SK TR**

(30) Priority: **16.12.2005 US 750834 P**
**05.12.2006 US 633665**

(43) Date of publication of application:
**27.08.2008 Bulletin 2008/35**

(73) Proprietor: **Cornell Research Foundation Inc.**
**Ithaca, NY 14850-1265 (US)**

(72) Inventors:
- **CHU, Chih-Chang**
**Ithaca, NY 14850 (US)**
- **LEWIS, Patti, Jo**
**Highland Park, New Jersey 08904 (US)**

(74) Representative: **Burford, Anthony Frederick**
**Beck Greener**
**Fulwood House**
**12 Fulwood Place**
**London**
**WC1V 6HR (GB)**

(56) References cited:
**EP-A- 1 614 789**
**WO-A-02/49536**
**WO-A-2007/024125**
**US-A1- 2003 168 756**
**US-A1- 2004 013 819**
**US-A1- 2004 054 410**
**US-A1- 2004 126 405**
**US-A1- 2004 126 405**
**US-A1- 2005 095 695**
**US-B1- 6 503 538**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 960 513 B1

## Description

### Technical Field

The present invention is directed to poly(ester-amide) structures fabricated for biomedical application.

### Background of the Invention

Biodegradable poly(ester-amide)s are known for use for administration of drugs admixed with or chemically linked thereto applied as a drug eluting film or coating and for use in the manufacture of medical devices. See WO 02/18477A2; U.S. Patent No. 6,503,538; and Katsarava, R., et al., Journal of Polymer Science, Part A, Polymer Chemistry 37, 391-407 (1999). They have not heretofore been fabricated into a form suitable for bum treatment, wound coverage, artificial skin, or scaffolds for tissue engineering.

US 2004/013819 discloses oriented hollow fiber webs comprising hollow fibers having an internal diameter of 10 nm to 50 μm and an outer wall constructed of metal-containing inorganic compounds, polymers and/or metals, in which the hollow fibers are preferentially oriented in one direction. The webs can be produced by coating oriented template fiber webs of degradable materials with nondegradable materials and then degrading the degradable materials by, for example, thermal methods. Exemplified coating materials include polyester amides.

US 2004/126405 discloses a three dimensional cell scaffold for use in cell transplantation and/or organ reconstruction including a biocompatible polymer formed from a plurality of fibers configured so as to form a non-woven three dimensional open celled matrix having a predetermined shape, a predetermined pore volume fraction, a predetermined pore shape, and a predetermined pore size, with the matrix having a plurality of connections between the fibers. The preferred pore size is from 0.5 to 100 microns, especially 1 to 50 microns. The polymers may be biodegradable or biostable or combinations thereof and specified polymers include polyester amides.

### Summary of the Invention

It has now been discovered that the field for biomedical application of biodegradable poly(ester-amide)s can be enlarged from the uses previously proposed, by fabricating the biodegradable poly (ester-amides) into non-woven fabric of electrospun fibers. For example, it has been discovered that the poly(ester amide)s as claimed in U.S. Patent No. 6,503,538 can be fabricated into useful non-woven fabrics by electrospinning.

In one embodiment herein, denoted the first embodiment, the invention is directed to a non-woven fabric consisting essentially of biodegradable electrospun poly(ester amide) for use for biomedical application, which is sterilizable and has an average fiber diameter ranging from 0.1 to 10 micrometer, e.g., 1.0 to 4.0 micrometer, a median pore size ranging from 0.1 to 100 micrometer, e.g., 2 to 100 micrometers, a surface area ranging from 100 to 300 $m^2/g$, e.g., 150 to 300 $m^2/g$, an average thickness ranging from 0.01 to 0.500 mm, e.g., 0.05 to 0.200 mm, a flexural rigidity ranging from 10 to 80 mg·cm, an average air permeability ranging from 10 to 100 $ft^3/min/ft^2$ (3 to 30 $m^3/min/m^2$) an average water vapor transmission rate ranging from 200 to 500 $g/m^2/24h$, a wettability contact angle ranging from 40 to 80 degrees, e.g., 50 to 80 degrees, tensile stress property ranging from 0.01 to 0.10 $kgf/mm^2$, tensile strain property ranging from 100 to 800 %, Young's modulus ranging from 0.20 to 20.0 MPa and tensile toughness ranging from 0.50 to 3.0 MPa.

As used herein the term "biodegradable" means capable of being broken down into innocuous products by various enzymes such as trypsins, lipases and lysosomes in the normal functioning of the human body and living organisms (e.g., bacteria) and/or water environment.

As used herein the term "biomedical application" means application to clinical medicine.

The poly(ester-amide) of the non-woven fabric is preferably one that can be solution electrospun into fibers.

In a preferred case of the first embodiment, the poly(ester amide) of the non-woven fabric has a reduced viscosity ranging from 1.0 to 2.0 dL/g, e.g., 1.2 to 2.0 dL/g and is selected from one or more subunits A, one or more subunits B, and combinations thereof, where the one or more subunits A have the structure

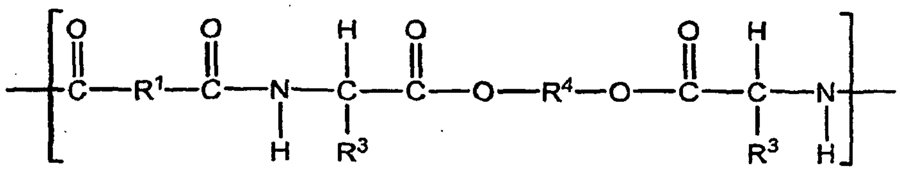

(I),

where $R^1$ is $(C_2-C_{20})$ alkylene, and where $R^3$ is hydrogen, $(C_1-C_{20})$ alkyl, $(C_2-C_6)$ alkenyl, $(C_2-C_6)$ alkynyl or $(C_6-C_{10})$ aryl $(C_1-C_6)$ alkyl;
and where the one or more subunits B have the structure

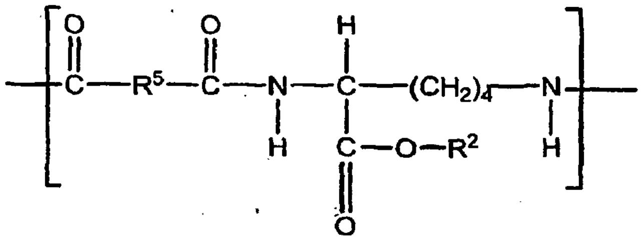

(II).

where $R^2$ is hydrogen or $(C_6-C_{10})$ aryl $(C_1-C_6)$ alkyl and where $R^5$ is $(C_2-C_{20})$ alkylene.

For purposes of scaffold for tissue engineering, the poly(ester-amide) of the non-woven fabric, in one case has the structure (I) where $R^1$ is $(CH_2)_8$, $R^3$ is

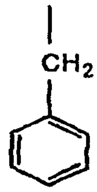

and $R^4$ is $C_4$-$C_8$ alkylene.

The poly(ester-amide) within the preferred case described above, determined to be most preferred of the poly (ester-amide)s tested, for a scaffold for tissue engineering, has the structure

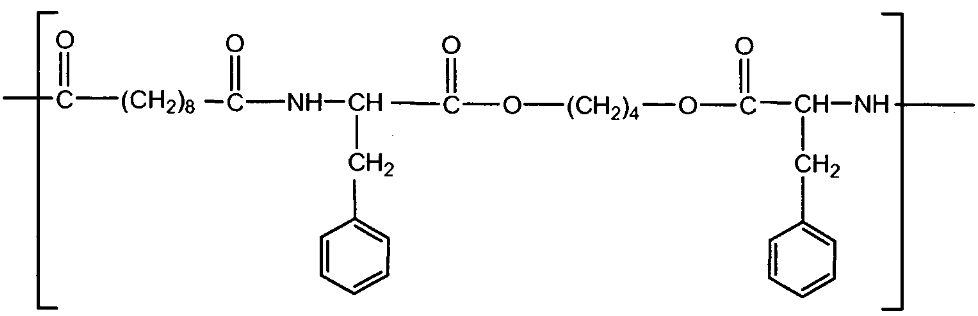

Suitability for a scaffold for tissue engineering is shown by cell attachment and proliferation in a Calcein-AM assay of cells seeded on the non-woven fabric. The seeding and assay for cell attachment and proliferation are described below. Calcein-AM is available from Molecular Probes (Eugene, OR). The cells used as model cells to show attachment and proliferation were keratinocytes, particularly Normal Human Epidermal Keratinocyte cell line (NHEK cell line), Catalog No. CC-2501 from Cambrex Bio Science Walkersville, Inc. (Walkersville, MD) or Biolife Solutions, Inc. (Oswego, NY). These cells were chosen for testing because of easy availability and ease of manipulation. This testing is relevant to use for a scaffold for tissue engineering because tissue engineering involves seeding of cells into a scaffold for proliferation into tissues.

Other utilities for the non-woven fabrics herein are bum treatment including adjunct therapy for burn treatment, wound coverage, partial thickness wound repair, healing acceleration, artificial skin, barrier to prevent tissue adhesions after surgery and administration of drugs or other agent physically or chemically associated therewith, for these purposes.

The non-woven fabrics herein arc advantageous over films of the same poly(ester-amide) in providing a three dimensional porous network structure that a two dimensional film does not have.

So far as scaffolds for tissue engineering are concerned, the non-woven fabrics herein are advantageous over films of the same poly(amide-ester) because they have a larger surface area for the cells to attach to and proliferate.

So far as utility for administration of drug or other agent is concerned; the drug or other agent is, e.g., matrixed into the non-woven fabric.

We turn now to determination of the various properties of the non-woven fabrics herein.

Sterilizability is determined as follows: Samples of nonwoven fabrics are cut to the size of the bottoms of wells of a 96-well microplate (1/4 inch; 0.63 cm). The samples are attached around the bottom edges with 15% poly(ester-amide) in chloroform solution. A microplate with samples therein is placed into a Medi-Plus ethylene oxide bag, the bag is sealed and air plasma sterilization is carried out using a Harrick Plasma Cleaner model PDC-32G (Harrick Scientific, NY) on high setting for five minutes.

Fiber diameter and pore size are determined as follows based on scanning electronmicroscopy (SEM) pictures using Scion Image for Windows (www.scioncorp.com/pages/scionimagewindows.htm): Pore size of nonwoven and average fiber diameter are measured from the SEM images using the Scion program. Electrospun fibrous poly(ester-amide) mats are sputter coated with gold for thirty seconds using a BAL-TEC sputter coater (Manchester, NH) (Bal-Tech SCD050), and the sputter coated mats are observed using a Hitachi S4500 (Mountain View, CA) scanning electron microscope and an accelerating voltage of 10kV.

Surface area is determined as follows: Surface area is analyzed using a Brunauer, Emmett and Teller (BET) surface area analyzer from Porous Materials, Inc. (Ithaca, NY). A poly(ester amide) sample is cut and weighed and is then placed in a BET tube. The test is run at -195.76°C with adsorbate nitrogen gas entering the system at 20 micrometer/minute under vacuum. One mat is used for providing all the samples for obtaining average surface area data.

Thickness is determined according to ASTM D1777-96 as follows: Measurements are carried out using a Sherman W. Frazier Compressometer using a circular, 9.525 mm diameter presser foot. Ten measurements are made at a pressure of 0.023 MPa (3.4 pounds per square inch) to obtain an average value.

Flexural rigidity is measured according to ASTM D1388 as follows: Standard commercially available spun bonded polypropylene (40 GMS, i.e., 40 gms per square meter) nonwoven samples are cut into 6 x 1 inch (15.25 x 2.5 cm) strips. Poly(ester amide) constructs are cut into 3 x 1 inch (7.6 x 2.5 cm) strips. The strips are mounted on a horizontal platform with one end sloping at a 45 degree angle. Each strip is slowly pushed off the platform in such a way that it overhangs. The fabric bends down and from the length and weight per square centimeter, the flexural rigidity G (stiffness) is calculated according to the following equation.

$$G = Wxc^3$$

where W = mg/cm$^2$ (the weight per unit area) and c is the bending length - the length of overhang in cm/2. The flexural rigidity of the spun bonded polypropylene is used as a control. The units are denoted with the terminology mg · cm. Five measurements are made to obtain an average value.

Air permeability is measured according to ASTM D737-96 as follows: Circular samples with a minimum diameter of 3.5 inches (8.9 cm) are mounted on a Frazier precision instrument (Silver Spring, MD). The rate of airflow through the fabric is measured under a differential pressure range of 0 - 1.0 inches of water (0 - 2.5 MPa). The data is expressed in ft$^3$ air/min/ft$^3$. The air permeability of spun bonded 40 GSM polypropylene nonwoven material is used as a control. Five measurements are made to obtain an average value.

Water vapor transmission rate is determined according to ASTM D6701-01 as follows: Samples are cut into 2.5 inch diameter circles and weighed to obtain sample density (gms/m$^2$) · 10mL of distilled water is placed into the bottom of model 305 water vapor permeability cups. Then the samples are mounted onto the cups. Each assembled system including cup, fabric and water is weighed at 0, 0.5, 1, 3, 5, 12, 24 and 48 hours. The test is performed at 21°C and 65% relative humidity. Three measurements are made to obtain an average value for water vapor transmission rate (water vapor permeability). The water vapor transmission rate is calculated according to the equation (G/t)A where G is the weight change in grams, t is the time during which G occurred, in hours, and A is the test area (cup's mouth area) in m$^2$. The results are expressed in grams moisture/m$^2$ fabric/24 hours. The water vapor transmission rate of spunbonded polypropylene 40 GSM nonwoven, is used as a control.

Wettability contact angle is determined as follows: Film of each poly(ester amide) is cast by pouring a layer of approximate thickness of 0.500 mm of 7% weight/weight, poly(ester-amide) in chloroform solution, onto a Teflon® plate. When a uniform thickness layer is obtained, the plate is covered by a watch glass to decrease evaporation rate. Each film layer is dried at room temperature for twenty-four hours. After the twenty-four hour drying time, each film is pulled from its Teflon® plate and cut into three 0.5 inch by 0.5 inch (1.25 x 1.25 cm) samples. The samples are mounted onto the contact angle analyzer (Hingham, MA) stage with double sided tape. Each stage is inserted into the analyzer and a small amount of distilled water or methylene iodide ($CH_2I_2$) is dropped onto the sample and brought into the viewing

area. The height and ½ the width of the droplet are measured and the contact angle is calculated according to the following equation:

$$\cos\theta = \frac{x^2 - y^2}{x^2 + y^2}$$

where
x =½ drop width, y = drop height, $\theta$ = contact angle.

Tensile properties are determined as follows: Tensile stress, tensile strain, Young's modulus and tensile toughness of poly(ester amide) and spunbonded 40 GSM polypropylene are measured. The samples are cut into 1 x 6 cm rectangular shapes and are mounted with vertical orientation in an Instron testing machine, model 1166. Tests are performed using a gauge length of 50 mm and a cross-head speed of 50 mm/minute. Average fabric thickness is used for calculating the tensile properties. The strength of a spunbonded polypropylene 50 GSM nonwoven material is used as a control. Five specimens are tested to obtain the average tensile properties.

Biodegradability is determined by *in vitro* $\alpha$-chymotrypsin catalyzed hydrolysis as described in Katsarava, R., et al., Journal of Polymer Science: Part A. Polymer Chemistry 37, 391-407 (1999).

Reduced viscosities are determined as follows:

Each poly(ester amide) (PEA) polymer is dissolved with m-cresol to a 0.25 g/dL concentration. After the PEA polymers are dissolved, the solution is poured into a model C572 Glass Cannon capillary viscometer. The capillary viscometer is placed into a VWR Scientific Model 1120 Constant Temperature Circulator and the temperature held constant at 25 degrees Celsius. Suction is applied to the solution until it is past the top mark. Once the solution flows past the mark, timed collection is started. The timed collection is ended when the solution passes the second mark. The procedure is repeated 5 times for the pure solvent (m-cresol) and each polymer solution. The reduced viscosity is calculated using the following equations.

$$\eta = \eta_s / \eta_p \qquad \text{Equation 1}$$

$\eta_s$ = solution time (seconds)
$\eta_p$ = m-Cresol time (seconds)

$$\eta_{sp} = \eta - 1 \qquad \text{Equation 2}$$

$$\eta_{reduced} = \eta_{sp} / \text{concentration } (0.25 \text{ g/dL}) \qquad \text{Equation 3}$$

Reference:

Jan F. Rabek, "Experimental methods in polymer chemistry", Wiley-Interscience, NY, 1980, Chapter 9 "Viscosimetric methods", pp. 123-136.

In another embodiment herein, denoted the second embodiment, the non-woven fabric of the first embodiment is made by a method comprising solution electrospinning of poly(ester-amide) and varying thickness of the non-woven fabric and/or solution concentration and/or collection distance and/or voltage and/or fiber diameter to vary pore size.

<u>Detailed Description</u>

The polymerizations to provide the poly(ester-amide)s can be carried out by an interfacial technique or by active

polymerization.

The poly(ester-amide)s described above, can be prepared by active polymerization as described in Katsarava, R., et al., Journal of Polymer Science: Part A: Polymer Chemistry 37, 391-407 (1999); U.S. Patent No. 6,503,538; and in WO 02/18477A2.

We turn now to the interfacial technique. This is described at pages 270-271 of Seymour/Carraher's Polymer Chemistry, Fifth Edition (2000). Description of the technique there includes the following "Many of the reactions can be carried out under essentially nonequilibrium conditions. The technique is heterophasic, with two fast-acting reactants dissolved in a pair of immiscible liquids, one of which is usually water. The aqueous phase typically contains the Lewis base - a diol, diamine or dithiol - along with any added base or other additive. The organic phase consists of a Lewis acid, such as an acid chloride, dissolved in suitable organic solvent, such as toluene, octane or pentane. Reaction occurs near the interface."

The poly(ester-amide)s made for testing herein are as follows:

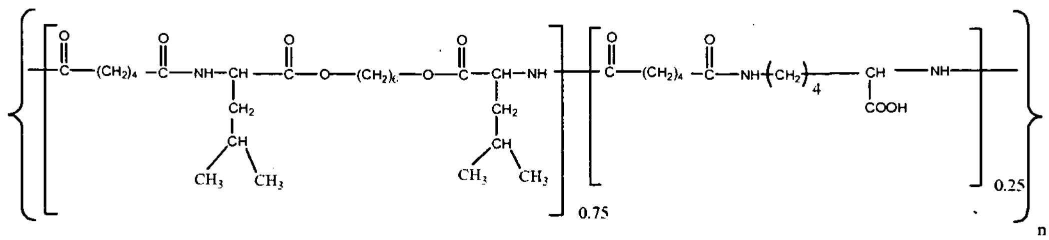

(GJ1)

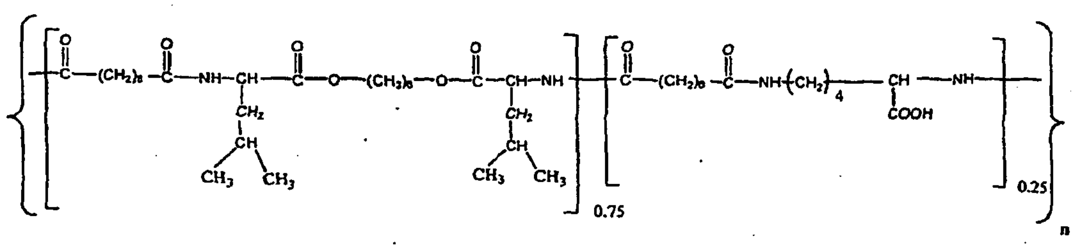

(GJ2)

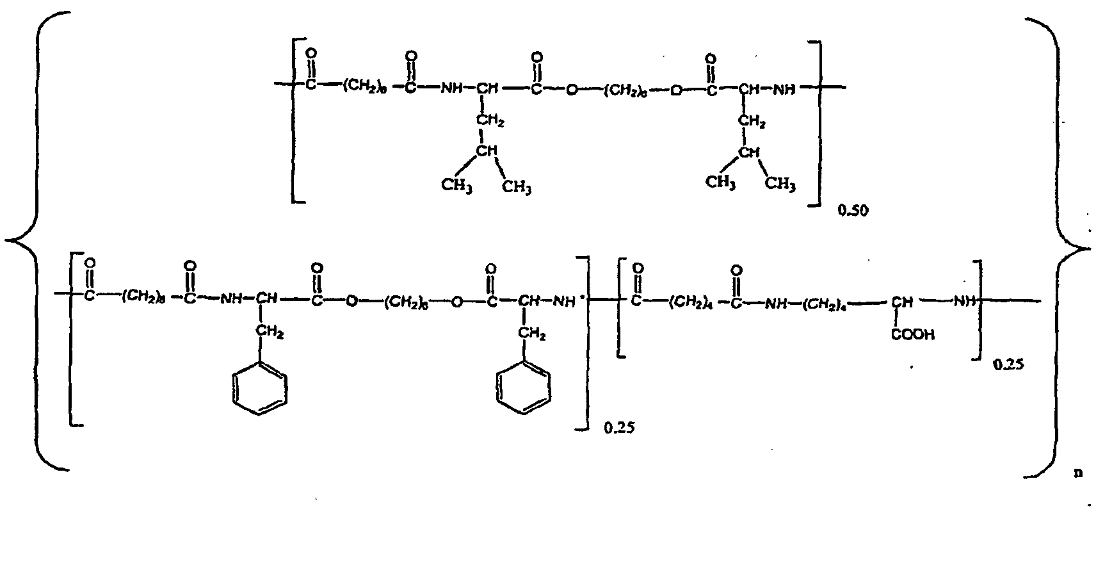

(GJ4)

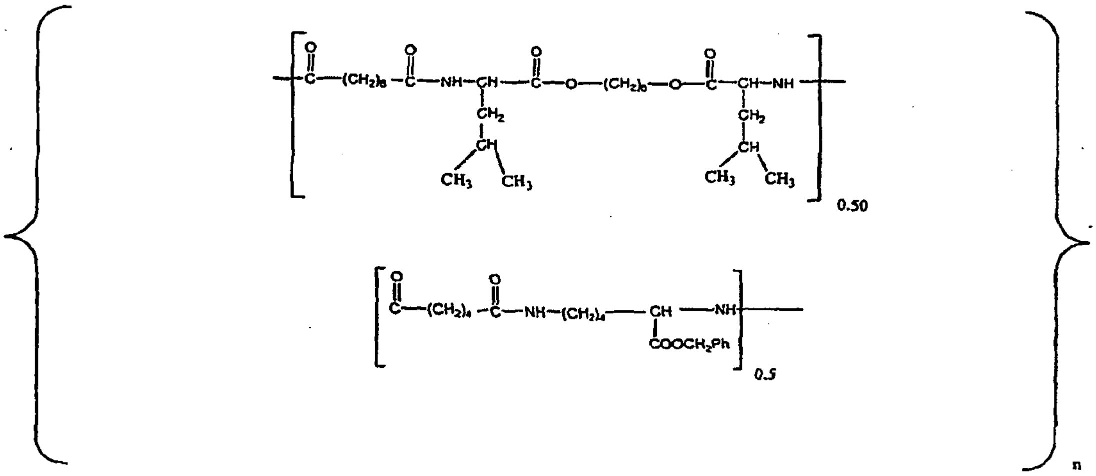

(GJ5)

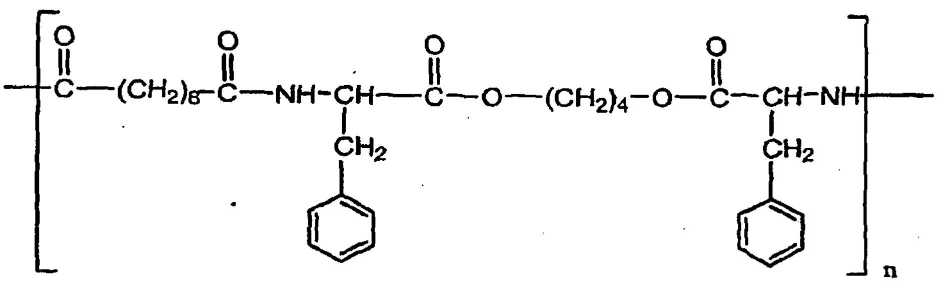

(8P4)

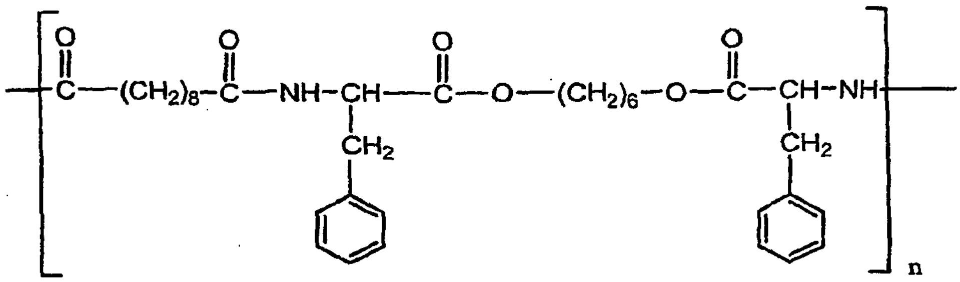

(8P6 ICP)

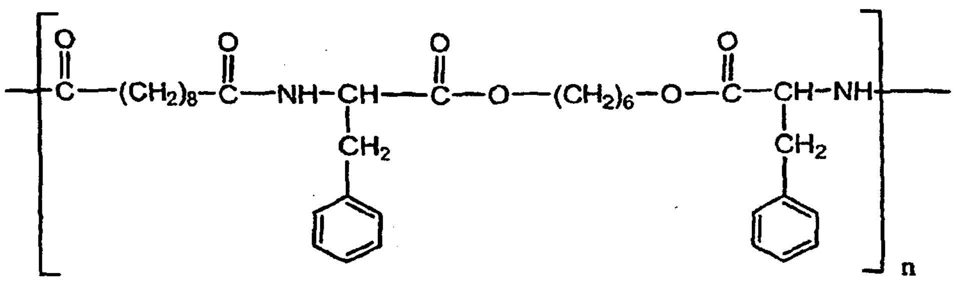

(8P6 ACP)

GJ1, GJ2, GJ4 and GJ5 were synthesized with the lysine unit being in the benzyl ester form. For GJ1 and GJ2, the benzyl ester of the lysine unit was more than 90% converted by hydrogenolysis to the free acid form for electrospin processing. For GJ4, 40% of the benzyl ester in the lysine unit was converted by hydrogenolysis to the free acid form

for electrospin processing. GJ5 was left entirely with the lysine unit in the benzyl ester form for electrospin processing.

GJ1, GJ2, GJ4 and GJ5 were made by active polymerization.

(8P4) and (8P6 ACP) were made by active polymerization.

(8P4 ICP) was made by the interfacial technique.

Reduced viscosities for the above are set forth in Table 1 below.

Table 1

| Structure | $\eta_{red}$ (dL/g) |
|---|---|
| GJ1 | 1.695 |
| GJ2 | 1.571 |
| GJ4 | 1.616 |
| GJ5 | 1.415 |
| 8P4 | 1.433 |
| SP6 ICP | 1.768 |
| 8P6 ACP | 1.554 |

The molecular weight can be determined from the reduced viscosities.

We turn now to electrospinning of the poly(ester-amide)s into fibers and formation of non-woven fabric.

Solution or melt electrospinning can be used.

Described below is the lab set up for the non-woven fabric production herein by solution electrospinning. However, any solution electrospinning system including conventional ones can be used.

For admixture of drug or other agent in the non-woven fabric, for example, drug or other agent that accelerates wound healing, the drug or other agent can be incorporated into polymer solution prior to solution electrospinning.

In the experiments carried out, polymer solutions were placed in a horizontally oriented 5cc glass syringe fitted with a 24 gauge blunt end needle. The collection plate was a wire mesh taped to three layers of wax paper on the collecting face. The wire mesh was connected to a grounding wire and was positioned 10-15 cm from the needle. The voltages applied to the needle ranged from 9 to 20 kV. Flow rates tried ranged from 0.01 mL/min to 0.10 mL/min. Preferred conditions determined were 0.02 mL/min flow rate, 15 cm distance between needle end and collection plate and 11kV voltage applied to the needle.

Droplets are formed at the needles end. The charge on the needle provides an electric charge in the droplets emitting therefrom to overcome the surface tension of a droplet to produce a jet of polymer giving rise to unstable flow toward the collecting plate manifested by a series of electrically induced bending instabilities/whipping motions and evaporation of solvent and production of elongated polymer fibers and deposit thereof on the wax paper of the collection plate as a non-woven fabric of the polymer.

The solvent selected for dissolving a poly(ester-amide) for the solution electrospinning should provide dissolution within 24 hours at room temperature and solution viscosity and evaporation rate suitable to produce fiber by solution electrospinning. A solution viscosity of 1-20 poise, a surface tension for the solution of 33-35 dyne/sm and a solvent evaporation rate of at leat 1.0 $g/m^2/h$ are aimed for.

Since comparative data was being determined, a solvent was sought that would dissolve all seven specific poly(ester-amide)s in 24 hours at room temperature. This criterion was found to be met by both dimethylformamide (DMF) and chloroform. Chloroform was selected for use in experiments because it produced higher viscosity poly(ester-amide) solutions compared to an equal concentration of the same poly(ester-amide) in DMF and a higher evaporation rate so that fibers would more likely solidify and dry before reaching the collection plate.

The most uniform poly(ester-amide) fibers were observed at 12.5, 15% and 17% concentration of poly(ester-amide) in chloroform and 15% and 17% were chosen for further test work.

GJ1 was not able to be solution electrospun. It wouldn't form fibers and the solution would just create spray droplets. However, it may be able to be melt electrospun.

GJ4 provided the best solution electrospinning results - a single thin filament pulled out of the droplet during electrospinning.

In the experiments carried out, the poly(ester-amide) non-woven fabrics obtained had average fiber diameter ranging from 2 to 4 micrometer, a median pore size of 50 micrometer, a surface area of 220 $m^2/g$, an average thickness of 0.1 mm, a flexural rigidity ranging from 4 to 65 mg·cm, an average air permeability ranging from 25 to 90 $ft^3/min/ft^2$ (7.5 to 27.5 $m^3/min/m^2$), an average water vapor transmission rate ranging from 280 to 430 $g/m^2/h$, a wettability contact angle ranging from 60 to 75 degrees, tensile stress property ranging from 0.035 to 0.095 $kgf/mm^2$, tensile strain property

ranging from 125 to 795%, Young's modulus ranging from 0.9 to 14.5 MPa and tensile toughness ranging from 0.90 to 2.10 MPa.

Pore size can be varied by varying thickness of the non-woven fabric. The greater the thickness, the smaller the pore size. Increasing the solution concentration causes increase in fiber diameter. Fiber diameter is related to collection distance. For example, in experiments carried out on poly(ester amide) 8P4, electrospun at 15% concentration in solution, use of a collection distance of 15 cm created fiber diameter of 2μm whereas use of a collection distance of 10 cm created a fiber diameter of 2.35 μm. At 20 cm, the fiber diameter becomes larger because the collection plate is beginning to be moved outside the electrical charge field resulting in lesser force to draw the fiber to its full extension. Thus the collection distance had an initial inverse effect on fiber diameter as the collection distance increased. Fiber diameter increases with voltage increase. In experiments carried out on poly(ester amide) 8P4 at 15% concentration in solution at voltage levels of 9, 11, 15 and 20 kV, as applied voltage was increased, the fiber diameter first is increased and then is decreased and number of bead defects increased with increasing voltage. Variation in fiber diameter can be used to vary porosity. For a given coverage, g/m$^2$, increase in fiber diameter can provide increase or decrease in pore size.

Testing for cell attachment and proliferation (tissue engineering scaffold utility) was carried out as follows:

Firstly, all seeding was carried out as follows: Normal human epidermal keratinocytes (NHEK cells) were plated in a monolayer in 75 cm$^2$ tissue culture flask and cultured till the cells reached three passages. After the third passage, the cells were removed by trypsin treatment, counted, and seeded onto the constructs at a density of 10,000 cells/well. The constructs were maintained in an incubator at 37°C with 5% $CO_2$ The medium was changed every three days.

Cell attachment as a result of seeding was determined as follows:

To assay the cells attached, the medium was removed and wells were rinsed with Hanks Balanced Salt Solution (HBSS) without $Mg^{2+}$, $Ca^{2+}$, and phenol red. The constructs were exposed to a Calcein-AM solution (1:250 HBSS without phenol red) for thirty minutes. Cell numbers were indicated directly by the relative fluorescence units (RFU) obtained from a Spectrafluor. Calcein-AM fluorescent pictures were obtained by Zeiss optical fluorescent microscope.

To determine cell proliferation, the cells were assayed at day 1, 3 and 7. A Spectrafluor and Zeiss optical fluorescent microscope collected the readings.

Of the poly(ester amide)s specifically described above, 8P4 was considered the model for nonwoven fabric production and the following results were obtained on nonwoven fabric from 8P4.

Results are set forth below:

For nonwoven fabric from 8P4 from electrospinning from 15% concentration at feed rate 0.02 mL/min, 11 kV, 10 cm collection distance, absorbencies (RFU) denoting cell proliferation were 14,000, 18,000 and 31,000 at days 1, 3 and 7 as compared to absorbencies of 22,000, 26,000 and 44,000 for a well with only NHEK cells and no scaffold.

The non-woven fabric from 8P4 from electrospinning at feed rate of 0.02 mL/min, 11 kV and 10 cm collection distance had properties as follows: Average fiber diameter of 3 micrometers, median pore size of 50 micrometers, a surface area of 220m$^2$/g, an average fabric thickness of 0.102 mm, a flexural rigidity of 19.5 mg · cm, an air permeability of 31.8 ft$^3$/min/ft$^2$ (9.69 m$^3$/min/m$^2$), an average water vapor transmission rate of 427 g/m$^2$/24h, a wettability content angle of 66.4 degrees, tensile stress of 0.073 kgf/mm$^2$, tensile strain of 144.5%, Young's modulus of 14.31 MPa, and tensile toughness of 0.902 MPa.

As indicated above, the nonwoven fabric herein can incorporate drug or other agent.

Examples of these are nonwoven fabric incorporating agent for accelerating wound healing or for burn treatment or adjunct therapy for burn treatment" e.g., gallium nitrate, for administration of nitroxyl radical (e.g., 2,2,6,6-tetramethylpiperidine-l-oxy radical), e.g., to reduce intimal hyperplasia in vascular grafts or to reduce tissue adhesion by retarding smooth muscle cell proliferation, or for administration of rapamycin (sirolimus) to prevent tissue adhesion after abdominal or other surgery, or for administration of therapeutic protein (as suggested by incorporation into the fabric of the model protein albumin).

A working example of incorporation of gallium nitrate into non-woven fabric within the scope of the invention follows:

Approximately 1.5g of the poly(ester amide) (8P4) was dissolved in 4g of chloroform ($CHCl_3$ 99.8% HPLC grade), purchased from Aldrich Chemical Co., Inc. Gallium (III) nitrate hydrate, purchased from Sigma-Aldrich Inc. was dissolved in 500 mg 99.8% anhydrous N,N-dimethylformamide (DMF) (Aldrich), in amounts of 10 and 500 mg. The

DMF-dissolved gallium nitrate was slowly added (droplet by droplet) to chloroform dissolved PEA without any visible precipitation, to provide 1.2, 1.0 and 0.2 grams per gram of poly(ester amide).

The homogeneous mixed drug (gallium nitrate)/polymer solution was electrospun at 15 kV under a steady flow rate of 0.025 mL/min using a spinneret with an orifice of diameter 0.2 mm as the jet with the distances of approximately 15-cm from the collecting plate. The electrospun fibers were collected in the form of thin fabric on a metal sheet (10 em X 15 cm) wrapped with wax paper. The fibrous fabric is peeled off the collecting wax paper.

In order to determine release profiles of gallium nitrate from the fabric, a calibration curve was prepared as follows: Solutions of known concentrations of gallium nitrate in chloroform (not completely dissolved) were extracted with 10-mL deionized water. The electrical resistance of the aqueous solutions from extraction was measured. A calibration curve was constructed based on conductivity (inverse of measured resistance) and known concentration of gallium nitrate.

Drug release was established at 1, 2, 3, 4, 5, 8, 12, 18 and 28 days by extraction with water and measuring conductivity and generating gallium nitrate release profiles for 1.2 grams of gallium nitrate per gram (8P4), 1.0 gram of gallium nitrate per gram (8P4) and 0.2 gm of gallium nitrate per gram (8P4). In each case there was a burst of drug release within the first 5 days. The drug release profile suggests that release time is independent of the concentration of gallium nitrate incorporation in the fiber.

The role of gallium nitrate in promoting wound healing is demonstrated by Staiano-Coico, L., J. Surgical Res. 103, 134-140 (2002).

Variations

The foregoing description of the invention has been presented describing certain operable and preferred embodiments. It is not intended that the invention should be so limited since variations and modifications thereof will be obvious to those skilled in the art, all of which are within the scope of the invention as defined in the following claims.

**Claims**

1. A non-woven fabric consisting essentially of biodegradable electrospun poly(ester amide) for use for biomedical application, which is sterilizable and has an average fiber diameter ranging from 0.1 to 10 micrometer, a median pore size ranging from 0.1 to 100 micrometer, a surface area ranging from 100 to 300 $m^2$/g, an average thickness ranging from 0.01 to 0.500 mm, a flexural rigidity ranging from 10 to 80 mg·cm, an average air permeability ranging from 3 to 30 $m^3/min/m^2$ (10 to 100 $ft^3/min/ft^2$), an average water vapor transmission rate ranging from 200 to 500 g)$m^2$/24h, a wettability contact angle ranging from 40 to 80 degrees, tensile stress property ranging from 0.01 to 0.10 kgf/$mm^2$, tensile strain property ranging from 100 to 800 %, Young's modulus ranging from 0.20 to 20.0 MPa and tensile toughness ranging from 0.50 to 3.0 MPa.

2. A non-woven fabric of Claim 1, wherein the poly(ester-amide) has an average fiber diameter ranging from 1.0 to 4.0 micrometer, a median pore size ranging from 2 to 100 micrometers, a surface area ranging from 150 to 300 $m^2$/g, an average thickness ranging from 0.05 to 0.200 mm, a flexural rigidity ranging from 10 to 80 mg·cm, an average air permeability ranging from 10 to 100 $ft^3/min/ft^2$ (3 to 30 $m^3/min/m^2$), an average water vapor transmission rate ranging from 200 to 500 g/$m^2$/24h, a wettability contact angle ranging from 50 to 80 degrees, tensile stress property ranging from 0.01 to 0.10 kgf/$mm^2$, tensile strain property ranging from 100 to 800 %, Young's modulus ranging from 0.20 to 20.0 MPa and tensile toughness ranging from 0.50 to 3.0 MPa.

3. A non-woven fabric of Claim 1 or Claim 2, wherein the poly(ester-amide) has been solution electrospun.

4. A non-woven fabric of any one of the preceding claims, wherein the poly(ester-amide) has a reduced viscosity ranging from 1.0 to 2.0 dL/g and is selected from one or more subunits A, one or more subunits B, and combinations thereof, wherein the one or more subunits A have the structure

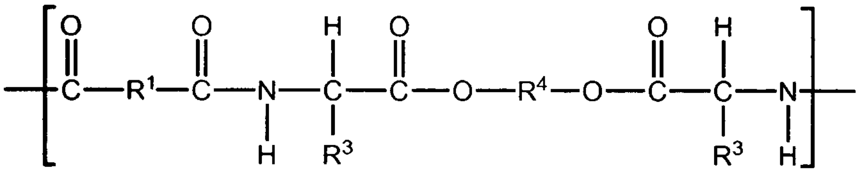

(I),

where $R^1$ is $(C_2-C_{20})$ alkylene, and where $R^3$ is hydrogen, $(C_1-C_{20})$ alkyl, $(C_2-C_6)$ alkenyl, $(C_2-C_6)$ alkynyl or $(C_6-C_{10})$ aryl $(C_1-C_6)$ alkyl;
and wherein the one or more subunits B have the structure

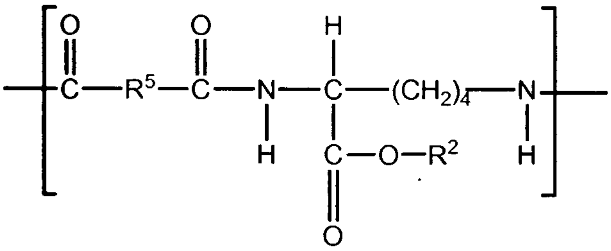

(II)

where $R^2$ is hydrogen or $(C_6-C_{10})$ aryl $(C_1-C_6)$ alkyl and where $R^5$ is $(C_2-C_{20})$ alkylene.

**5.** A non-wovcn fabric of any one of the preceding claims, wherein the poly(ester-amide) is selected from those of the structure:

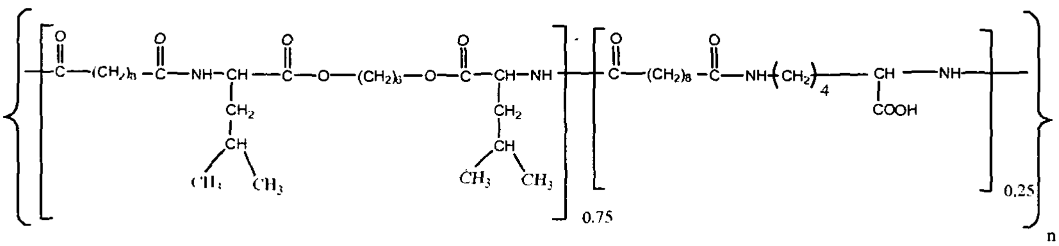

(GJ2)

;

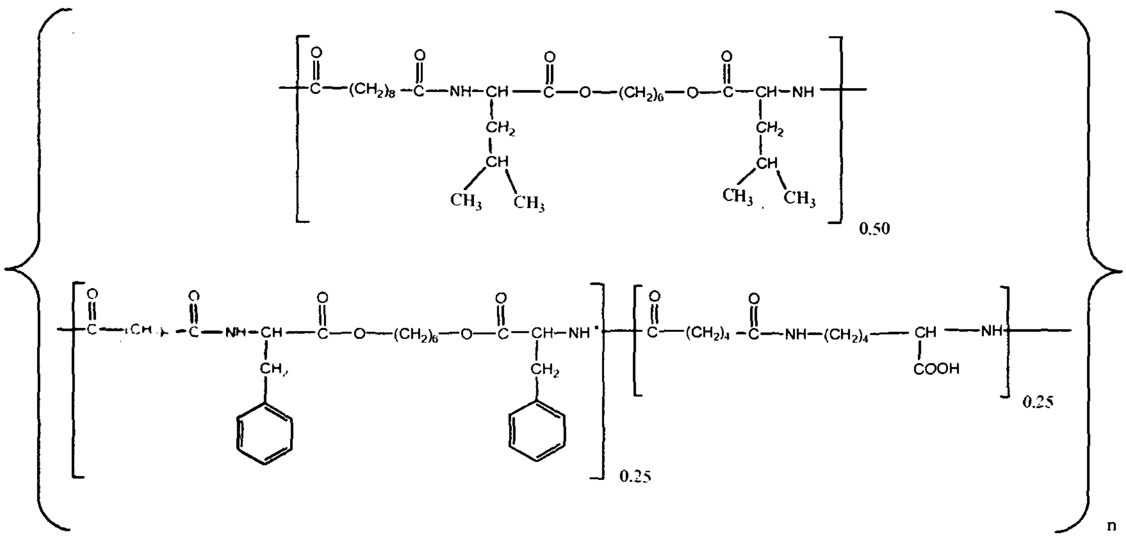
(GJ4) ;

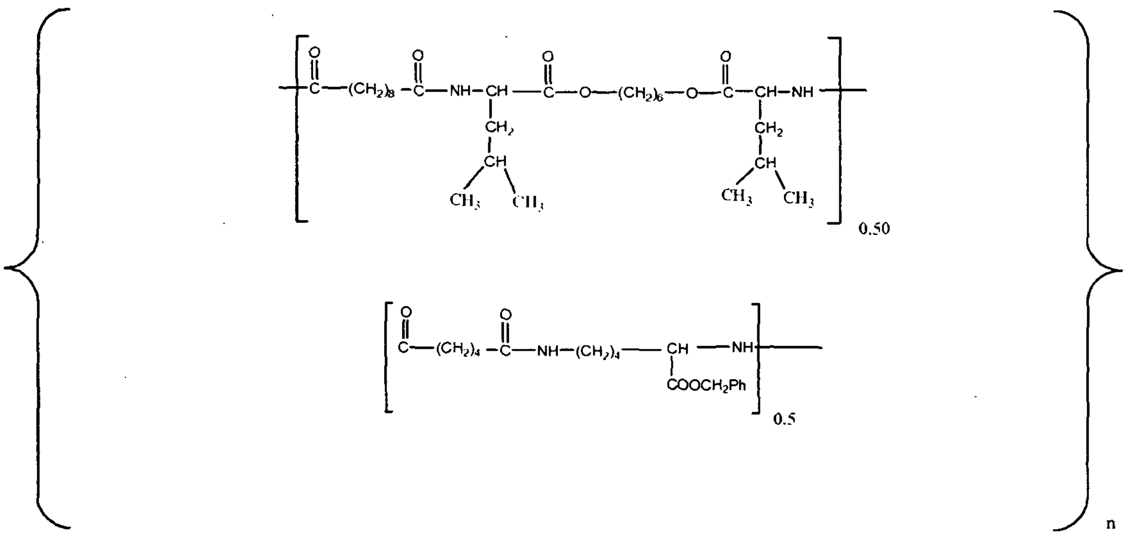
(GJ5) ;

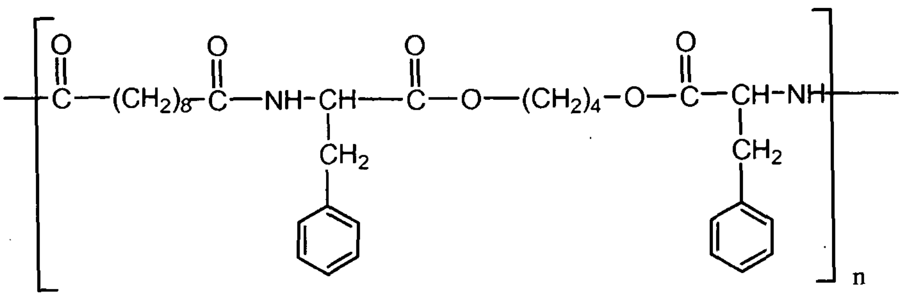
(8P4) ;

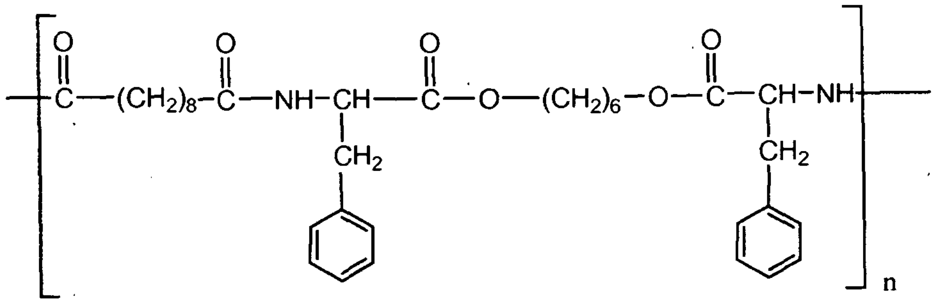

(8P6 ICP)

;

and

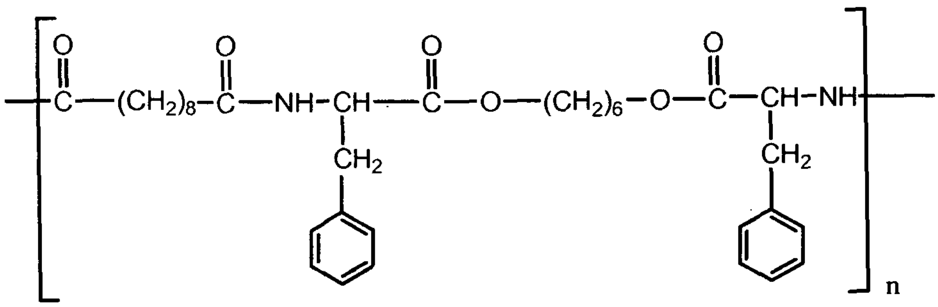

(8P6 ACP)

.

6. A non-woven fabric of Claim 4, wherein the polyester amide has the structure (I) where $R^1$ is $(CH_2)_8$, $R^3$ is

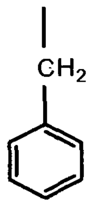

and $R^4$ is $C_4$-$C_8$ alkylene.

7. A non-woven fabric of Claim 6, wherein the polyester amide has the structure:

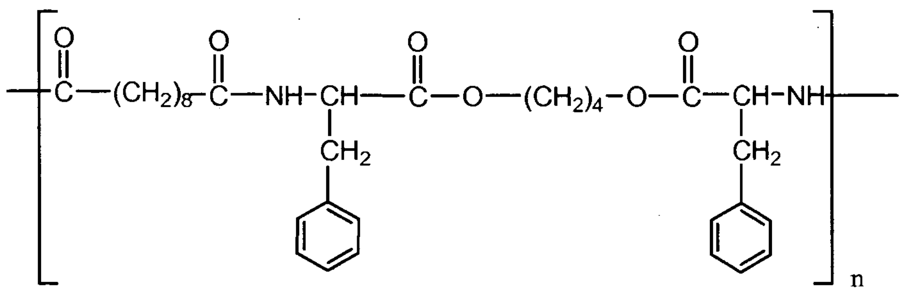

(8P4)

8. A non-woven fabric of any one of the preceding claims which when seeded with NHEK cells shows cell attachment and proliferation in a Calcein-AM assay.

**9.** A non-woven fabric of any one of the preceding claims with a drug or other agent that accelerates healing, matrixed therein.

**10.** A method for making a non-woven fabric of any one of the preceding claims comprising solution electrospinning of poly(ester-amide) and varying thickness of the non-woven fabric and/or solution concentration and/or collection distance and/or voltage and/or fiber diameter to vary pore size.

**11.** A non-woven fabric of any one of Claims 1 to 9 for use as a scaffold for tissue engineering.

**12.** A non-woven fabric of any one of Claims 1 to 9 for use in bum treatment.

**13.** A non-woven fabric of Claim 12, wherein said treatment is selected from adjunct therapy for bum treatment, wound coverage, partial thickness wound repair, healing acceleration, artificial skin, barrier to prevent tissue adhesions after surgery and administration of drugs or other agent physically or chemically associated therewith, for these purposes.

**Patentansprüche**

**1.** Faservlies bestehend im Wesentlichen aus biologisch abbaubarem elektrogesponnenem Poly(esteramid) zur Verwendung für eine biomedizinische Anwendung, das sterilisierbar ist und einen mittleren Faserdurchmesser im Bereich von 0,1 bis 10 μm, eine mittlere Porengröße im Bereich von 0,1 bis 100 μm, eine Oberflächenfläche im Bereich von 100 bis 300 $m^2$/g, eine mittlere Dicke im Bereich von 0,01 bis 0,500 mm, eine Biegesteifigkeit im Bereich von 10 bis 80 mg*cm, eine mittlere Luftdurchlässigkeit im Bereich von 3 bis 30 $m^3$/min/$m^2$ (10 bis 100 $ft^3$/min/$ft^2$), eine mittlere Wasserdampf-Durchlassrate im Bereich von 200 bis 500 g/$m^2$/24h, einen Benetzungskontaktwinkel im Bereich von 40 bis 80 Grad, eine Zugspannungseigenschaft im Bereich von 0,01 bis 0,10 kgf/$mm^2$, eine Zugbeanspruchungseigenschaft im Bereich von 100 bis 800 %, einem Youngschen Modul im Bereich 0,20 bis 20,0 MPa, und eine Zugzähigkeit im Bereich von 0,50 bis 3,0 MPa hat.

**2.** Faservlies nach Anspruch 1, wobei das Poly(esteramid) einen mittleren Faserdurchmesser im Bereich von 1,0 bis 4,0 μm, eine mittlere Porengröße im Bereich von 2 bis 100 μm, eine Oberflächenfläche im Bereich von 150 bis 300 $m^2$/g, eine mittlere Dicke im Bereich von 0,05 bis 0,200 mm, eine Biegesteifigkeit im Bereich von 10 bis 80 mg*cm, eine mittlere Luftdurchlässigkeit im Bereich von 10 bis 100 $ft^3$/min/$ft^2$ (3 bis 30 $m^3$/min/$m^2$), eine mittlere Wasserdampf-Durchlassrate im Bereich von 200 bis 500 g/$m^2$/24h, einen Benetzungskontaktwinkel im Bereich von 50 bis 80 Grad, eine Zugspannungseigenschaft im Bereich von 0,01 bis 0,10 kgf/$mm^2$, eine Zugbeanspruchungseigenschaft im Bereich von 100 bis 800 %, einen Youngschen Modul im Bereich von 0,20 bis 20,0 MPa, und eine Zugzähigkeit im Bereich von 0,50 bis 3,0 MPa hat.

**3.** Faservlies nach Anspruch 1 oder Anspruch 2, wobei das Poly(esteramid) lösungsgesponnen ist.

**4.** Faservlies nach einem der vorangehenden Ansprüche, wobei das Poly(esteramid) eine reduzierte Viskosität in dem Bereich von 1,0 bis 2,0 dL/g hat und ausgewählt ist aus einer oder mehreren Untereinheiten A, einer oder mehreren Untereinheiten B, und Kombinationen davon, wobei die eine oder mehreren Untereinheiten A die Struktur

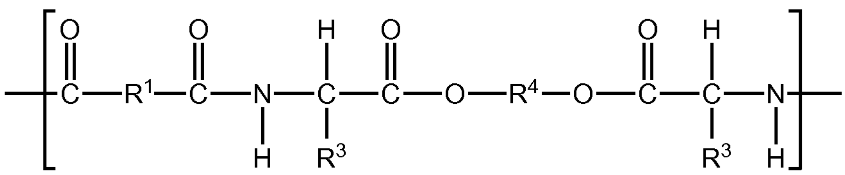

(I),

haben, wobei $R^1$ ($C_2$-$C_{20}$)-Alkylen ist, und wobei $R^3$ Wasserstoff, ($C_1$-$C_{20}$)-Alkyl, ($C_2$-$C_6$)-Alkenyl, ($C_2$-$C_6$)-Alkinyl oder ($C_6$-$C_{10}$)-Aryl-($C_1$-$C_6$)-alkyl ist;
und wobei die eine oder mehreren Untereinheiten B die Struktur

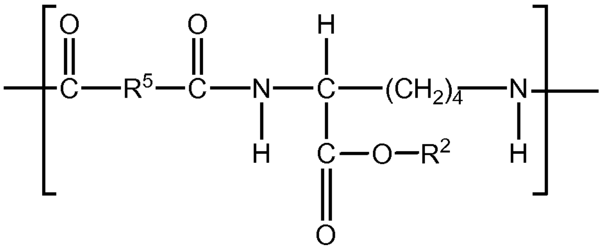

(II)

haben, wobei $R^2$ Wasserstoff oder $(C_6\text{-}C_{10})$-Aryl-$(C_1\text{-}C_6)$-alkyl ist und wobei $R^5$ $(C_2\text{-}C_{20})$-Alkylen ist.

**5.** Faservlies nach einem der vorangehenden Ansprüche, wobei das Poly(esteramid) ausgewählt ist aus denjenigen der Struktur:

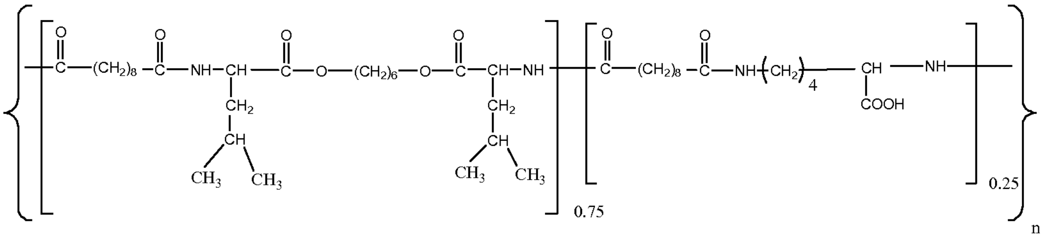

(GJ2)

;

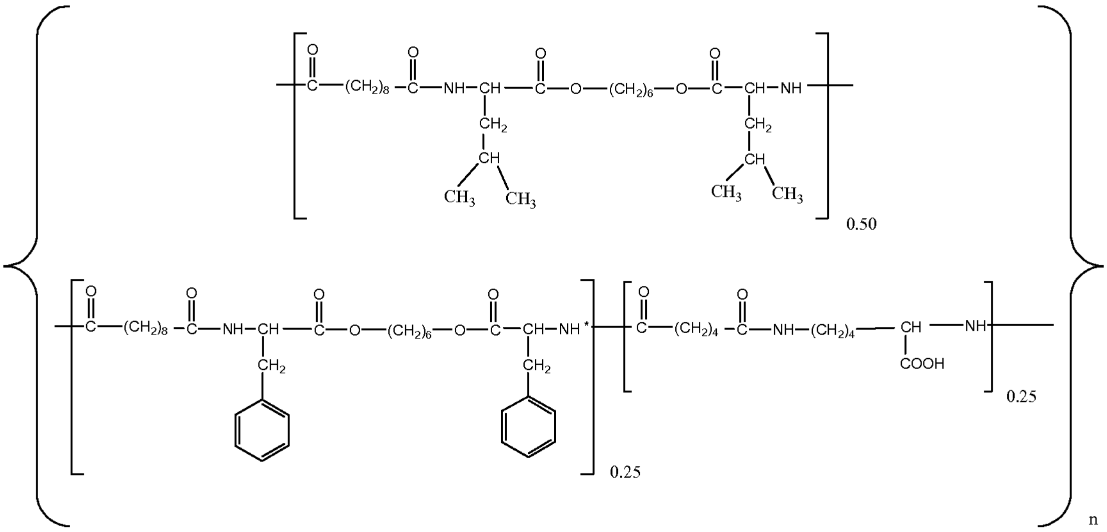

(GJ4)

;

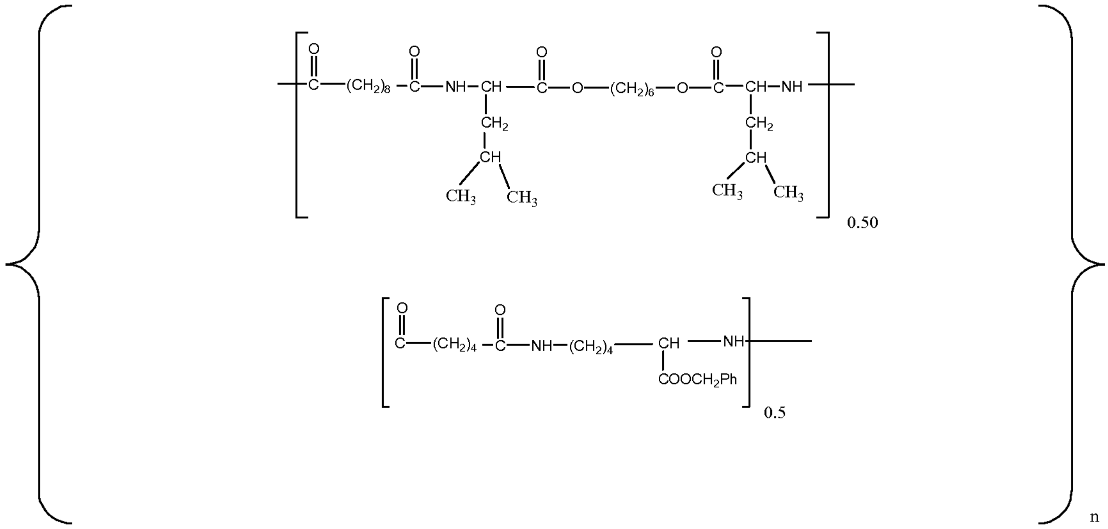

(GJ5)

;

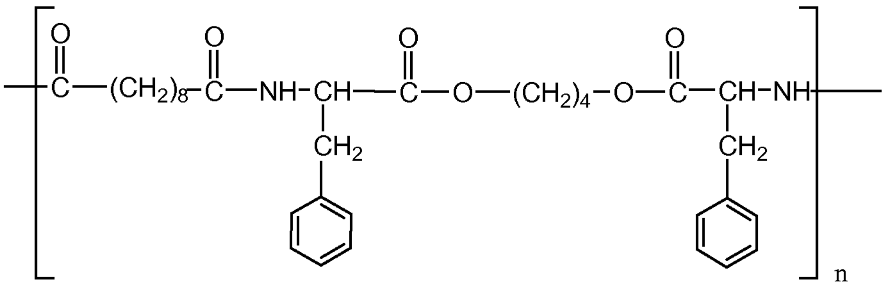

(8P4)

;

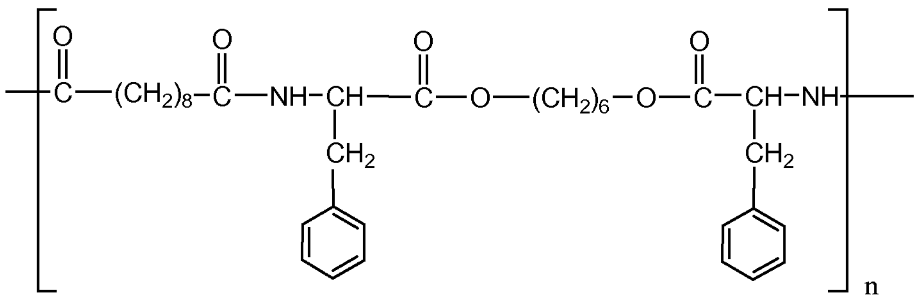

(8P6 ICP)

;

und

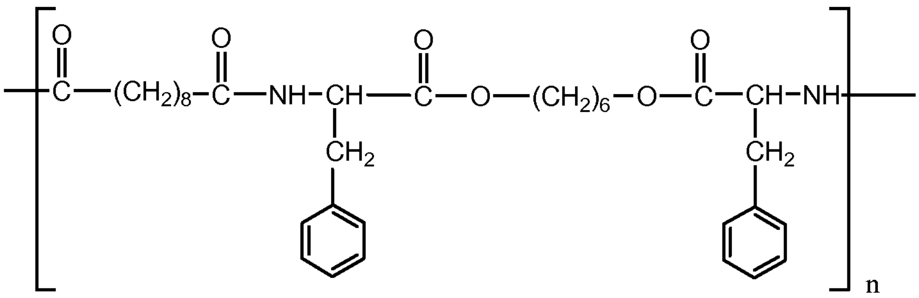

(8P6 ACP)

.

6. Faservlies nach Anspruch 4, wobei das Polyesteramid die Struktur (I) hat, wobei $R^1$ $(CH_2)_8$ ist, $R^3$

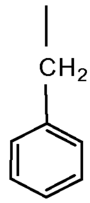

ist, und $R^4$ $C_4$-$C_8$-Alkylen ist.

7. Faservlies nach Anspruch 6, wobei das Polyesteramid die Struktur

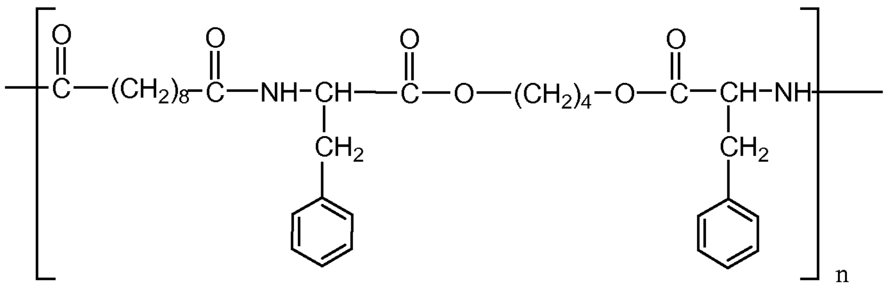

(8P4)

hat.

8. Faservlies nach einem der vorangehenden Ansprüche, das, wenn es mit NHEK-Zellen beimpft ist, in einem Calcein-AM-Assay Zell-Anhaftung und -Proliferation zeigt.

9. Faservlies nach einem der vorangehenden Ansprüche, als Matrix mit einem darin eingebetteten Arzneimittel oder anderen Mittel, das eine Heilung beschleunigt.

10. Verfahren zur Herstellung eines Faservlieses nach einem der vorangehenden Ansprüche, aufweisend Lösungs-Elektrospinnen von Poly(esteramid) und Variieren der Dicke des Faservlieses und/oder der Lösungskonzentration und/oder der Sammeldistanz und/oder der Spannung und/oder des Faserdurchmessers, um die Porengröße zu variieren.

11. Faservlies nach einem der Ansprüche 1 bis 9 zur Verwendung als ein Gerüst zur Gewebekonstruktion.

12. Faservlies nach einem der Ansprüche 1 bis 9 zur Verwendung bei der Behandlung von Verbrennungen.

13. Faservlies nach Anspruch 12, wobei die Behandlung ausgewählt ist aus einer Unterstützungstherapie zur Behandlung von Verbrennungen, Wundabdeckung, Teildicken-Wundreparatur, Beschleunigung der Heilung, künstlicher Haut, Sperre zur Verhinderung von Gewebe-Adhäsionen nach einem chirurgischen Eingriff und Verabreichung von Arzneimitteln oder einem anderen Mittel, das physikalisch oder chemisch damit in Verbindung steht, für diese Zwecke.

## Revendications

1. Non-tissé essentiellement constitué de poly(ester-amide) biodégradable électrofilé pour une utilisation destinée à une application biomédicale, lequel est stérilisable et présente un diamètre moyen de fibres de 0,1 à 10 μm, une taille moyenne de pores de 0,1 à 100 μm, une surface spécifique de 100 à 300 $m^2$/g, une épaisseur moyenne de 0,01 à 0,500 mm, une rigidité à la flexion de 10 à 80 mg.cm, une perméabilité moyenne à l'air de 3 à 30 $m^3$/min/$m^2$ (10 à 100 $pieds^3$/min/$pieds^2$), une vitesse de transmission moyenne de la vapeur d'eau de 200 à 500 g/$m^2$/ 24 h, un angle de contact de mouillabilité de 40 à 80 degrés, des propriétés de contrainte de traction de 0,01 à 0,10 kgf/$mm^2$, des propriétés de déformation à la traction de 100 à 800 %, un module de Young de 0,20 à 20,0 MPa et une résistance à la traction de 0,50 à 3,0 MPa.

2. Non-tissé selon la revendication 1, dans lequel le poly(ester-amide) présente un diamètre moyen de fibres de 1,0 à 4,0 μm, une taille moyenne de pores de 2 à 100 μm, une surface spécifique de 150 à 300 $m^2$/g, une épaisseur moyenne de 0,05 à 0,200 mm, une rigidité à la flexion de 10 à 80 mg.cm, une perméabilité moyenne à l'air de 10 à 100 $pieds^3$/min/$pieds^2$ (3 à 30 $m^3$/min/$m^2$), une vitesse de transmission moyenne de la vapeur d'eau de 200 à 500 g/$m^2$/24 h, un angle de contact de mouillabilité de 50 à 80 degrés, des propriétés de contrainte de traction de 0,01 à 0,10 kgf/$mm^2$, des propriétés de déformation à la traction de 100 à 800 %, un module de Young de 0,20 à 20,0 MPa et une résistance à la traction de 0,50 à 3,0 MPa.

3. Non-tissé selon la revendication 1 ou la revendication 2, dans lequel le poly(ester-amide) a été électrofilé en solution.

4. Non-tissé selon l'une quelconque des revendications précédentes, dans lequel le poly(ester-amide) présente une viscosité réduite de 1,0 à 2,0 dL/g et est choisi parmi de une ou plusieurs sous-unités A, une ou plusieurs sous-unités B et des combinaisons de celles-ci, dans lequel la une ou les plusieurs sous-unités A présentent la structure

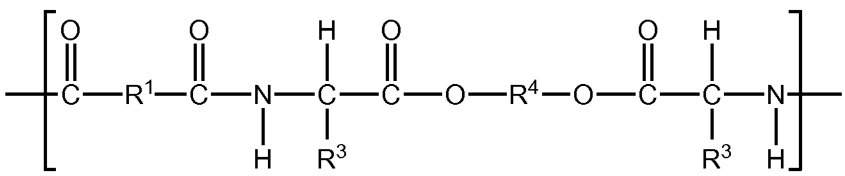

(I),

où $R^1$ est un groupe alkylène en $C_2$-$C_{20}$ et où $R^3$ est l'hydrogène, un groupe alkyle en $C_1$-$C_{20}$, alcényle en $C_2$-$C_6$, alcynyle en $C_2$-$C_6$ ou (aryle en $C_6$-$C_{10}$)-(alkyle en $C_1$-$C_6$) ;
et dans lequel la une ou les plusieurs sous-unités B présentent la structure

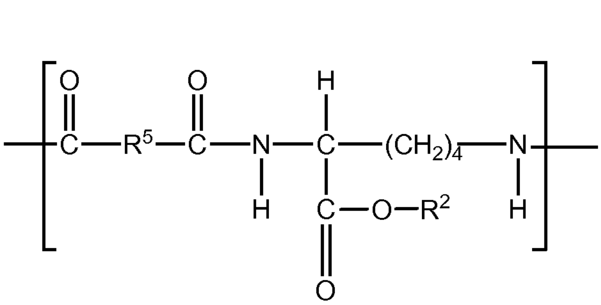

(II)

dans laquelle $R^2$ est l'hydrogène ou un groupe (aryle en $C_6$-$C_{10}$)-(alkyle en $C_1$-$C_6$) et où $R^5$ est un groupe alkylène en $C_2$-$C_{20}$.

5. Non-tissé selon l'une quelconque des revendications précédentes, dans lequel le poly(ester-amide) est choisi parmi

ceux de la structure :

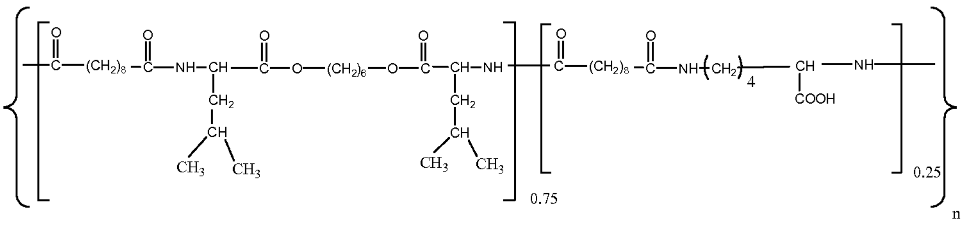

(GJ2)

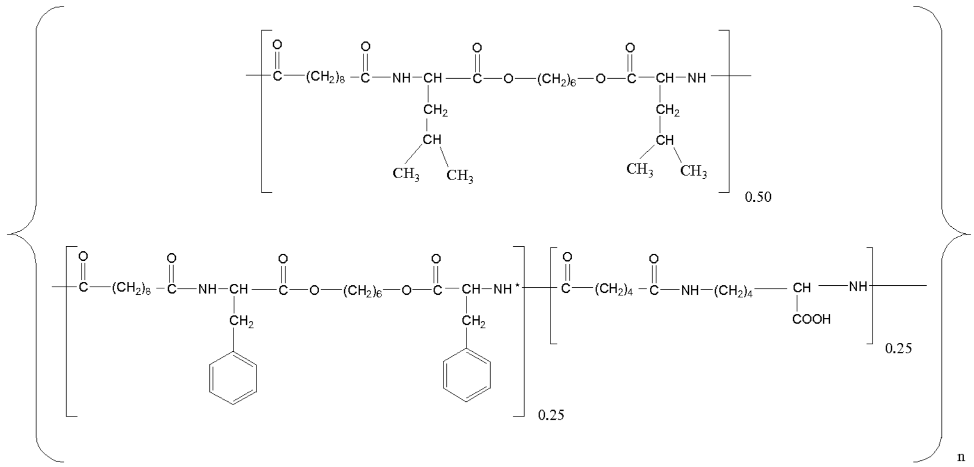

(GJ4)

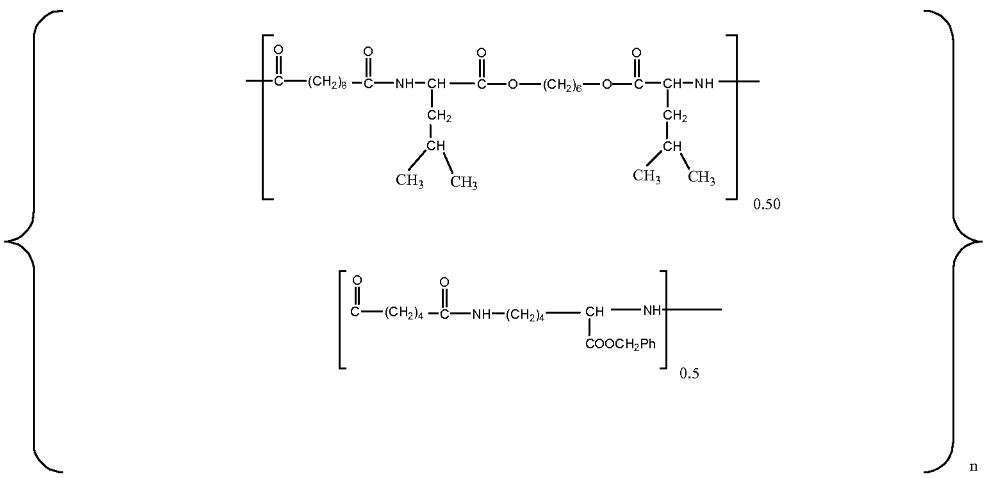

(GJ5)

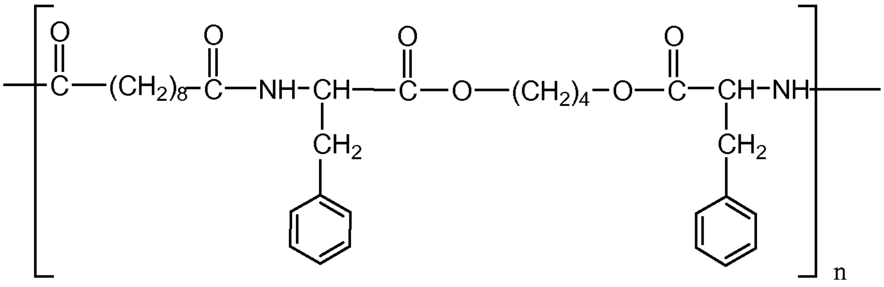

(8P4)

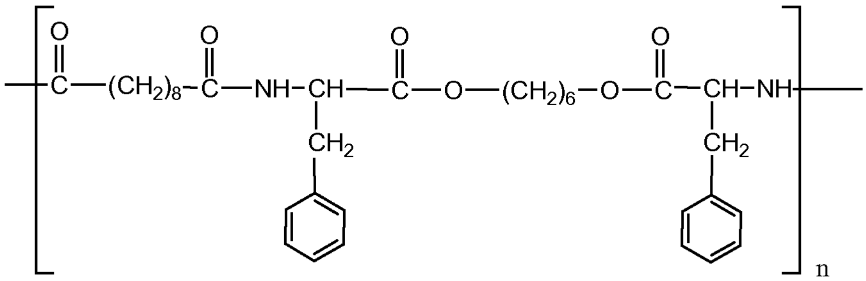

(8P6 ICP)

;

et

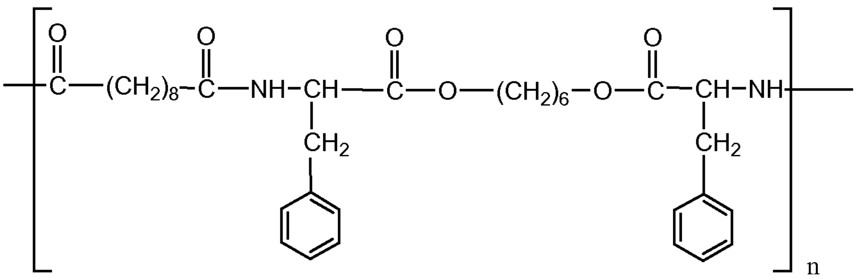

(8P6 ACP)

**6.** Non-tissé selon la revendication 4, dans lequel le polyester-amide présente la structure (I) où $R^1$ est $(CH_2)_8$, $R^3$ est

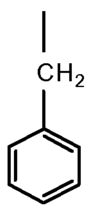

et $R^4$ est un groupe alkylène en $C_4$-$C_8$.

**7.** Non-tissé selon la revendication 6, dans lequel le polyesteramide présente la structure :

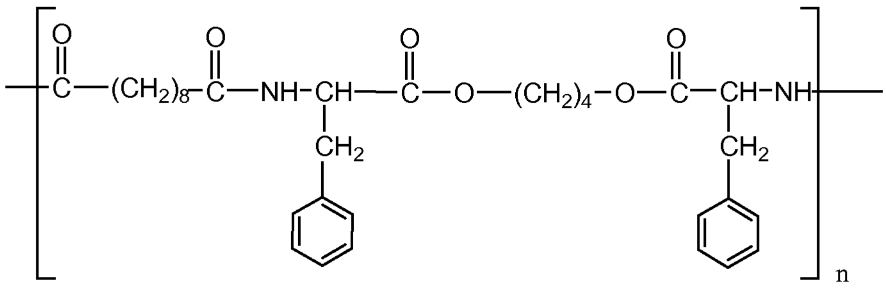

(8P4)

**8.** Non-tissé selon l'une quelconque des revendications précédentes, lequel lorsqu'il est ensemencé avec des cellules NHEK présente une fixation des cellules et une prolifération dans un dosage Calcein-AM.

**9.** Non-tissé selon l'une quelconque des revendications précédentes avec un médicament ou un autre agent qui accélère la guérison, mis en matrice dans celui-ci.

**10.** Procédé de fabrication d'un non-tissé selon l'une quelconque des revendications précédentes comprenant l'électrofilage en solution de poly(ester-amide) et la variation de l'épaisseur du non-tissé et/ou de la concentration de solution et/ou de la distance de recueil et/ou de la tension et/ou du diamètre de fibre pour faire varier la taille de pores.

**11.** Non-tissé selon l'une quelconque des revendications 1 à 9 pour une utilisation comme échafaudage pour la conception de tissus.

**12.** Non-tissé selon l'une quelconque des revendications 1 à 9 pour une utilisation dans le traitement des brûlures.

**13.** Non-tissé selon la revendication 12, dans lequel ledit traitement est choisi parmi une thérapie d'appoint pour le traitement de brûlures, la couverture de plaies, la réparation de plaies en épaisseur partielle, l'accélération de guérison, la peau artificielle, la barrière pour éviter les adhérences de tissus après une chirurgie et l'administration de médicaments ou d'autres agents physiquement ou chimiquement associés à ceux-ci, à ces fins.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0218477 A2
- US 6503538 B
- US 2004013819 A
- US 2004126405 A


### Non-patent literature cited in the description


- **Katsarava, R. et al.** *Journal of Polymer Science, Part A, Polymer Chemistry,* 1999, vol. 37, 391-407
- **Katsarava, R. et al.** *Journal of Polymer Science: Part A. Polymer Chemistry,* 1999, vol. 37, 391-407
- Viscosimetric methods. **Jan F. Rabek.** Experimental methods in polymer chemistry. Wiley-Interscience, 1980, 123-136
- **Katsarava, R. et al.** *Journal of Polymer Science: Part A: Polymer Chemistry,* 1999, vol. 37, 391-407
- **Staiano-Coico, L.** *J. Surgical Res.,* 2002, vol. 103, 134-140